# EUROPEAN PATENT SPECIFICATION

(11) **EP 1 412 503 B1**
(45) Date of publication and mention of the grant of the patent: **31.12.2008**
(21) Application number: 02749112.5
(22) Date of filing: 02.08.2002
(51) Int. Cl.: C12N 15/62, C12N 15/12, C07K 14/715, C12N 15/24, C07K 14/54, G01N 33/68, A61K 38/20, A61K 48/00, A61P 31/18, A61P 37/00, A61P 19/02

(54) **A FUSION PROTEIN**
EIN FUSIONSPROTEIN
PROTEINE HYBRIDE

(30) Priority: 03.08.2001 GB 0119015
(43) Date of publication of application: 28.04.2004
(73) Proprietor: University College Cardiff Consultants Ltd., Cardiff, Wales CF24 0DE (GB)
(72) Inventor: JONES, Simon Arnett Cardiff School of Biosciences, Cardiff CF10 3US (GB); TOPLEY, Nicholas Institute for Nephrology, Cardiff CF14 4XN (GB)
(74) Representative: Tombling, Adrian George
(86) International application number: PCT/GB2002/003581
(87) International publication number: WO 2003/014359

(56) References cited:
- EP-A- 1 094 081
- WO-A-99/02552
- FISCHER M ET AL: "A BIOACTIVE DESIGNER CYTOKINE FOR HUMAN HEMATOPOIETIC PROGENITOR CELL EXPANSION" NATURE BIOTECHNOLOGY, NATURE PUBLISHING, US, vol. 15, no. 2, 1 February 1997 (1997-02-01), pages 142-145, XP002047603 ISSN: 1087-0156 cited in the application
- JOSTOCK T ET AL: "Immunoadhesins of interleukin-6 and the IL-6/soluble IL-6R fusion protein hyper-IL-6" JOURNAL OF IMMUNOLOGICAL METHODS, ELSEVIER SCIENCE PUBLISHERS B.V.,AMSTERDAM, NL, vol. 223, no. 2, 4 March 1999 (1999-03-04), pages 171-183, XP004158716 ISSN: 0022-1759 cited in the application
- KOLLET O ET AL: "THE SOLUBLE INTERLEUKIN-6 (IL-6) RECEPTOR/IL-6 FUSION PROTEIN ENHANCES IN VITRO MAINTENANCE AND PROLIFERATION OF HUMAN CD34+CD38-/LOW CELLS CAPABLE OF REPOPULATING SEVERE COMBINED IMMUNODEFICIENCY MICE" BLOOD, W.B. SAUNDERS, PHILADELPHIA, VA, US, vol. 94, no. 3, 1 August 1999 (1999-08-01), pages 923-931, XP000972856 ISSN: 0006-4971 cited in the application
- CHEBATH J ET AL: "Interleukin-6 receptor-interleukin-6 fusion proteins with enhanced interleukin-6 type pleiotropic activities" EUROPEAN CYTOKINE NETWORK, JOHN LIBBEY EUROTEXT LTD., MONTROUGE, FR, vol. 8, no. 4, 1 December 1997 (1997-12-01), pages 359-365, XP002087946 ISSN: 1148-5493 cited in the application
- HORIUCHI S ET AL: "HIGH-LEVEL PRODUCTION OF ALTERNATIVELY SPLICED SOLUBLE INTERLEUKIN-6 RECEPTOR IN SERUM OF PATIENTS WITH ADULT T-CELL LEUKAEMIA/HTLV-I-ASSOCIATED MYELOPATHY" IMMUNOLOGY, BLACKWELL SCIENTIFIC PUBLICATIONS, GB, vol. 95, no. 3, November 1998 (1998-11), pages 360-369, XP009010710 ISSN: 0019-2805
- MUELLER-NEWEN G ET AL: "PURIFICATION AND CHARACTERIZATION OF THE SOLUBLE INTERLEUKIN-6 RECEPTOR FROM HUMAN PLASMA AND IDENTIFICATION OF AN ISOFORM GENERATED THROUGH ALTERNATIVE SPLICING" EUROPEAN JOURNAL OF BIOCHEMISTRY, BERLIN, DE, vol. 236, no. 3, 1996, pages 837-842, XP001152510 ISSN: 0014-2956
- JONES SIMON A ET AL: "The soluble interleukin 6 receptor: Mechanisms of production and implications in disease." FASEB JOURNAL, vol. 15, no. 1, January 2001 (2001-01), pages 43-58, XP002246850 ISSN: 0892-6638

## Description

The present invention relates to a fusion protein comprising a functional IL-6 molecule and a functional DS-sIL-6R molecule. The present invention also relates to a nucleic acid encoding the fusion protein, methods for producing the fusion protein and the use of the fusion protein in the treatment of infectious diseases and inflammatory and immunological disorders.

Interleukin-6 (IL-6) is a major inflammatory cytokine, which is responsible for regulating a variety of cellular events including proliferation/differentiation, hematopoiesis and regulation of immune responses. Activation of these processes is regulated by the binding of IL-6 to a specific receptor (IL-6R) which is found on the surface of certain cells. Since the presence of IL-6R is confined to only a small number of cell types the activity of IL-6 itself is limited. Binding to a soluble form of the IL-6R (sIL6R) can however modulate the biological activities of IL-6. The sIL-6R has been identified in a variety of bodily fluids and is elevated in numerous diseases. The sIL-6R is able to bind IL-6 and facilitate activation of cell types that would not normally respond to IL-6 alone. Consequently, control of sIL-6R release is an important process in the regulation of IL-6 activities.

Soluble cytokine receptors are typically generated through either proteolytic cleavage (PC) or differential mRNA splicing (DS). In the case of sIL-6R, both mechanisms are utilised and result in the generation of two isoforms, which are herein termed PC-sIL-6R and DS-sIL-6R Although both forms are structurally related, DS-sIL-6R is distinguished from that of PC-sIL-6R by the addition of 10 unique amino acids at its proximal COOH-terminal tail. Consequently, two distinct forms of sIL-6R control the overall properties of this soluble receptor.

As indicated above, many of the biological activities assigned to interleukin-6 (IL-6) are mediated through its ability to bind sIL-6R (Jones et al., FASEB. J. 15, 43-58, 2001). Indeed, formation of a sIL-6R/IL-6 complex has been shown to stimulate a variety of cellular responses that include cellular proliferation, differentiation and regulation of inflammatory events. Activation of these processes is achieved by interaction of the stimulatory complex of the ubiquitously expressed membrane-bound gp 130, which acts as a universal signal-transducing subunit for all IL-6-related cytokines (Heinrich et al., Eur. J. Biochem. 236, 837-842, 1998). Consequently, the sIL6R-IL-6 complex acts as an agonist of cell types that although express gp130, would not inherently respond to IL-6 itself. Given that the cellular expression of the cognate IL-6R is largely confined to hepatocytes and leukocyte sub-population, sIL-6R has the capacity to widen the range of cell types that are responsive to IL-6.

The identification of elevated sIL-6R levels in numerous clinical conditions has emphasized the potential for this soluble receptor to regulate both local and systemic IL-6-mediated responses (Jones *et al.,* 2001 (*supra*)). As a result it is essential that the cellular events controlled by IL-6 itself be distinguished from those mediated via the sIL6R/IL-6 complex. Central to this issue has been the necessity to ascertain how sIL-6R release is regulated *in vivo.* Understanding the mechanisms which control sIL-6R levels is however confounded by the presence of the two isoform, PC-sIL-6R or DS-sIL-6R. Although both forms are structurally related, the differentially spliced variant can be distinguished from the shed isoform by a novel proximal COOH-terminal sequence (GSRRRGSCGL) which is introduced as a consequence of the splicing process (Horiuchi et al., Immunology 95, 360-369, 1998). To date, it is unclear why there are two isoforms to control the activities of sIL-6R.

Since PC- and DS-sIL-6R might individually coordinate the overall properties of sIL-6R *in vivo,* it is essential to consider their temporal relationship during the progression of an inflammatory event, whilst also assessing their ability to elicit individual cellular events. Examination of clinical samples from various disease states have previously confirmed that release of each isoform is differentially regulated and depends upon the age of the individual, the disease state studied and the stage of the disease progression (Jones *et al.,* 2001 *(supra),* Horiuchi *et al.,* 1998 *(supra)* and Muller-Newen et al., Eur. J. Biochem. 236, 837-842, 1996).

Within the last 4-5 years it has been shown that HIV enters cells through utilizing the cell surface protein CD4, and one of two distinct co-receptors that have been identified as CXCR4 (for T-trophic strains of HIV) and CCR5 (for M-trophic strains of HIV). The natural function of these co-receptors is to act as chemokine receptors and MIP-1α, MIP-β and RANTES all bind to CCR5. Indeed, HIV patients who possess significantly elevated circulating levels of these chemokines are less likely to develop full blown AIDS than those individuals who have lower levels of MIP-1α, MIP-1β and RANTES. Since all 3 chemokines as well as M-trophic strains of HIV bind CCR5, high levels of MIP-1α, MIP-1β and RANTES compete with the virus for CCR5 binding and effectively suppress HIV entry. Consequently, any factor capable of redressing the balance of this competition in the favour of the chemokine can be useful as an HIV therapy.

In Fischer et al., (Nat. Biotechnol., 15, 142-5, 1997) a fusion protein comprising IL-6 linked by a linker to PC-sIL-6R is shown to be a potent stimulator of early haematopoietic precursors. The fusion protein is termed hyper-IL-6 (H-IL-6) by those skilled in the art. The use of H-IL-6 is also described in Jostock et al., (J. Immunol. Methods, 223, 171-183, 1999), Kollet et al., (Blood, 94, 923-931, 1999) and Chebath et al., (Eur. Cytokine. Netw., 8 359-65, 1997). In WO 00/01731 and WO 99/02552 a fusion protein comprising IL-6 linked to PC-s-IL-6R is disclosed.

In US patent US-A-5,919,763, H-IL-6 is said to have been used in the treatment of liver injury by promoting regeneration of the liver and its functions.

None of the prior art documents disclose or suggest a protein comprising IL-6 and DS-s-IL-6R.

The present invention provides a fusion protein comprising a functional IL-6 molecule and a functional DS-sIL-6R molecule, wherein the protein increases the expression of one or more of MIP-1α, MIP-1β, RANTES and IP-10.

MIP-1α is also referred to as CCL3; MIP1β is also referred to as CCL4, RANTES is also referred to as CCLS; and IP-10 is also referred to as CXCL10.

The present invention is based on the unexpected finding that a fusion protein comprising a functional IL-6 molecule and a functional DS-sIL-6R molecule results in the increased expression of one or more MIP-1α, MIP-1β, RANTES and IP-10. A fusion protein comprising a function IL-6 molecule and a PC-sIL-6R function molecule (i.e. H-IL-6) does not result in the increased expression of MIP-1α, MIP-1β, RANTES or IP-10. The fusion protein of the present invention therefore differs substantially in its function from H-IL-6. Both the fusion protein of the present invention and H-IL-6 have been found to increase the expression of MCP-1, but only the fusion protein of the present invention increases the expression of one or more of MIP-1α, MIP-1β, RANTES and IP-10. The fusion protein of the present invention may also increase the expression of other chemokines such as MIG (CXCL9) or ITAC (CXCL11).

The term "a functional IL-6 molecule" refers to any IL-6 molecule which functions in combination with DS-sIL-6R to increase the expression of MIP-1α, MIP-1β, RANTES or IP-10. In order to determine whether a candidate molecule is a functional IL-6 molecule, the candidate molecule can be tested in combination with DS-sIL-6R in order to determine whether there is an increase in expression of MIP-1α, MIP-1β, RANTES or IP-10. A suitable method for determining such function is described in Example 1 herein. Preferably, the functional IL-6 molecule comprises the sequence shown in SEQ ID NO.9

The term "a functional DS-sIL-6R molecule" refers to any DS-sIL-6R molecule which functions in combination with IL-6 to increase the expression of MIP-1α, MIP-1β, RANTES or IP-10. In order to determine whether a candidate molecule is a functional DS-sIL-6R molecule, the candidate molecule can be tested in combination with IL-6 in order to determine whether there is an increase in MIP-1α, MIP-1β, RANTES or IP-10. A suitable method for determining such function is described in Example 1 herein. Preferably, the functional DS-sIL-6R molecule comprises residues 113 to 364 of SEQ ID NO. 10 . It is further preferred that the functional DS-sIL-6R molecule comprises the sequence in SEQ ID NO. 10.

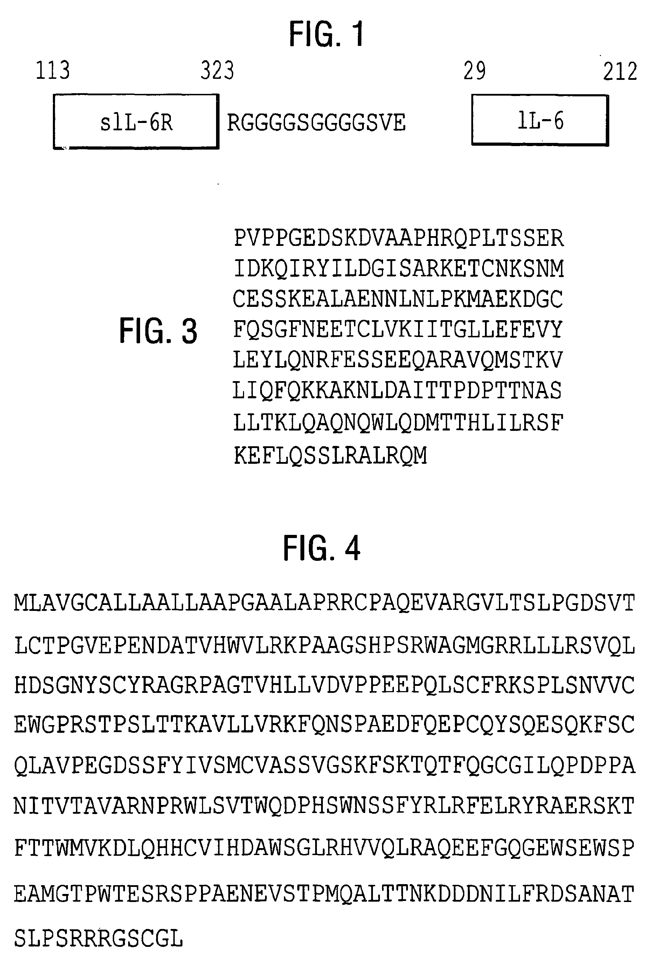

Modifications can be made which increase the function of the DS-sIL-6R functional molecule (i.e. increase the level of expression of MIP-1α, MIP-1β, RANTES or IP-10) above that achieved when using the DS-sIL-6R molecule having the sequence given in Figure 4. Suitable modifications may include increasing the length of the arginine run in the C-terminal 10 amino acids. Other suitable modifications may be random amino acid substitutions especially alanine substitutions, and truncation of the C-terminal 10 amino acids. Such modifications can be tested using the methods described herein. In particular RT-PCR has generated cDNA encoding for DS-sIL-6R. This cDNA molecule can be used as a template to modify the GSRRRGSCGL sequence or any other sequence of DS-sIL-6R. Olignucleotide primers based on this sequence can be used in PCR approaches to introduce novel codons within the proximal DS-sIL-6R sequence. This will allow generation of cDNA fragments that when expressed will result in serial truncation of the DS-sIL-6R COOH termini and the stepwise conversion of the DS-sIL-6R sequence to PC-sIL-6R. A QuikChangeTM site directed to mutagenesis kit (Stratagene) can also be used to modify individual residues within the GSRRRGSCGL sequence to pinpoint amino acids responsible for mediating DS-sIL-6R activity. All variants will be sequenced and cloned into a suitable vector, such as pVL1393 for baculovirus expression in SF9 cells (Horiuchi, 1998 (*supra*)) and pcDNA-3 for transient/stable expression in COS-7 cells (Elson et al., 2000, Nature Neuroscience, 3: 867-872). Other mutations which increase the function of DS-sIL-6R can also be made.

To ensure receptor functionality and to evaluate the binding kinetics of each DS-sIL-6R mutant, surface plasmon resonance technology can be used with IL-6 or soluble gp130 (sgp130) (for example in a 10 mg/ml coating stock) immobilised to a matrix such as CM5 carboxymethyl dextran. To identify residues important for eliciting the differential DS-sIL-6R activities, chemokine expression (MIP-1α, MIP-1β, RANTES and IP-10) by human peritoneal mesothelia cells (HPMC) will be monitored using ELISA (Hurst et al., Immunity, 14, 705-714, 2001). Luciferase reporter assays can also be used to determine whether the modification disrupts activation of RANTES promoter by DS-sIL-6R as described in the Materials and Methods below.

The functional IL-6 molecule and functional DS-sIL-6R molecule can be joined together via a linker or can be directly bound to each other by covalent linkages. Suitable flexible linkers are well known to those skilled in the art. Preferably, the flexible linker has the sequence GGGGSGGGGSLE. Alternatively, the linker can be in the form of a leucine zipper.

Methods for directly binding the functional IL-6 molecule to the functional DS-sIL-6R molecule can be easily determined from following the teaching in International Patent Application WO 00/01731, wherein a fusion protein comprising IL6 directly fused to PC-sIL-6R is disclosed.

The fusion protein of the present invention can comprise more than one functional IL-6 molecule and more than one functional DS-sIL-6R molecule. The ratio of IL-6 molecules to DS-sIL-6R molecules does not have to be 1:1 but is preferably 1:1. Preferably, the fusion protein according to the present invention comprises one functional IL-6 molecule and one functional DS-sIL-6R molecule.

It is particularly preferred that the fusion protein according to the present invention increases the expression of MIP-1α, MIP-1β, RANTES or IP-10 by at least 5 fold, preferably at least 10 fold, more preferably at least 15 fold and most preferably at least 25 fold. The increase in the expression of MIP-1α, MIP-β, RANTES or IP-10 can be measured *in vivo* or *in vitro.* Preferably the increase is measured in a suitable cell system such as a human peritoneal mesothelial cells (HPMC) grown *in vitro.* Suitable methods for measuring the increase in MIP-1α, MIP-1β, RANTES or IP-10 are disclosed herein.

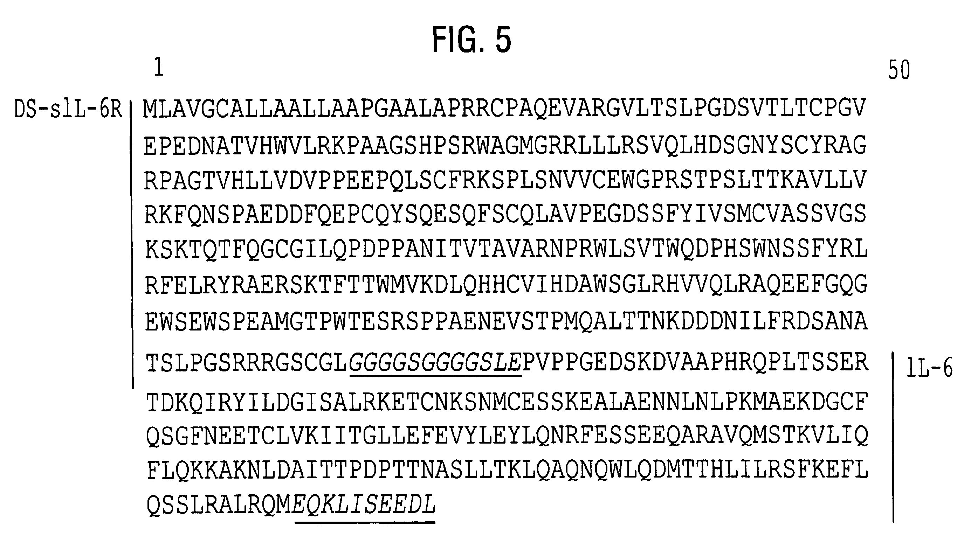

In a particularly preferred embodiment of the present invention, the fusion protein of the present invention has the sequence given in figure 5. The fusion protein given in figure 5 encodes a functional IL-6 molecule and a functional DS-sIL-6R molecule linked together by a flexible linker, with a C-terminal c-myc tag, which has been underlined. The c-myc tag is used to help with purification of the fusion protein and is preferably not part of the fusion protein of the present invention, especially when the fusion protein is used in a screening or medical context.

The present invention also provides a nucleic acid molecule encoding the fusion protein of the present invention.

The nucleic acid of the present invention can be obtained by methods well known in the art. For example, naturally occurring sequences may be obtained by genomic cloning or cDNA cloning from suitable cell lines or from DNA or cDNA derived directly from the tissues of an organism such as a human or mouse. Alternatively, the sequences can be synthesized using standard synthesis methods such as the phosphoramidite method.

Numerous techniques may be used to alter the nucleic acid sequence obtained by the synthesis or cloning procedures. Such techniques are well known to those skilled in the art. For example, site directed mutagenesis, or oligonucleotide directed mutagenesis and PCR techniques may be used to alter the DNA sequence. Such techniques are well known to those skilled in the art and are described in a vast body of literature known to those skilled in the art.

The present invention also provides an expression vector comprising the nucleic acid of the present invention. Expression vectors are well known for expressing nucleic acids in a variety of different organisms, including mammalian cells, insect cells, bacteria and eukaryotic microorganisms such as yeasts. All such expression vectors are well known to those skilled in the art and the use of expression vectors in order to express the nucleic acid sequence is a standard technique well known to those skilled in the art. Preferably the expression vector is a baculovirus expression vector.

Preferably the expression vector of the present invention comprises a promoter and the nucleic acid molecule of the present invention. The vector leads to the production of the fusion protein of the present invention. It is further preferred that the vector comprises any other regulatory sequences required to obtain expression of the nucleic acid molecule.

The nucleic acid molecule of the present invention may be expressed intracellularly in a suitable host cell. The promoter sequence may be directly linked to the nucleic acid molecule of the present invention in which case the amino acid at the N terminus of the encoded protein would be methionine encoded by the start ATG codon.

Alternatively, the fusion protein encoded by the nucleic acid molecule of the present invention can be secreted from a suitable host cell by linking a nucleotide sequence encoding a leader sequence to the nucleic acid molecule of the present invention. The encoded protein will comprise a leader sequence fragment and the fusion protein encoded by the nucleic acid molecule of the present invention. The leader sequence will lead to the secretion of the fusion protein out of the cell. Preferably there are processing sites between the lead sequence and the fusion protein encoded by the nucleic acid molecule of the present invention allowing the leader sequence to be cleaved off enzymatically or chemically. An example of such a leader sequence is the adenovirus triparite leader.

Preferably, the vector of the present invention comprises a promoter and other regulatory sequences required in order to obtain the desired expression of the nucleic acid molecule of the present invention.

The present invention also provides a host cell transformed with the vector of the present invention.

The term "transformation" refers to the insertion of an exogenous nucleic acid molecule into a host cell, irrespective of the method used for insertion, for example direct uptake, transduction, f-mating or electroporation. The exogenous nucleic acid may be obtained as a non-integrating vector (episome), or maybe integrated into the hosts genome.

Preferably the host cell is a eukaryotic cell, more preferably a mammalian cell, such as Chinese hamster ovary (CHO) cells, HPMCs, HeLa cells, baby hamster kidney (BKH) cells, cells of hepatic origin such as HepG2 cells, and myloma or hybridoma cell lines. Alternatively, the host cell is a prokaryotic cell such as *E. coli.*

The present invention further provides a method for producing the fusion protein of the present invention comprising transfecting a host cell with the vector of the present invention, culturing the transfected host cell under suitable conditions in order to lead to the expression of the nucleic acid molecule and production of the fusion protein of the present invention. The fusion protein may then by harvested from the transfected cells or from the cell growth media, depending on whether the fusion protein is secreted, using standard techniques.

The present invention also provides a screening method for identifying antagonists or agonists of the fusion protein of the present invention. The screening method preferably comprises testing a candidate molecule to determining if the presence of the candidate molecule affects the function of the fusion protein of the present invention. For example, the candidate molecule may increase or decrease the production of chemokines such as MCP-1α, MCP-1β, RANTES or IP-10, or alter the range of chemokines affected by the fusion protein.

Candidate molecules may be isolated from cells, cell-free preparations, chemical libraries, or natural product mixes. The candidate molecule may be a natural or modified substrate, ligand, enzyme, receptor, antibody molecule or structural or functional mimetic. For a review of suitable screening techniques, see Coligan et al., Current Protocols in Immunology, 1(2): Chapter 5, (1991).

The present invention also provides a pharmaceutical composition comprising the fusion protein of the present invention, the nucleic acid of the present invention or the expression vector according to the present invention, in combination with a pharmaceutically acceptable carrier, adjuvant or vehicle.

Suitable, pharmaceutically acceptable carriers, adjuvants or vehicles are discussed below.

The present invention also provides the fusion protein according to the present invention, the nucleic acid according to the present invention or the expression vector according to the present invention, for use in therapy.

The present invention also provides the use of the fusion protein according to the present invention, the nucleic acid according to the present invention or the expression vector according to the present invention in the manufacture of a medicament for the treatment or prophylaxis of bacterial peritonitis.

The present invention also provides the use of the fusion protein according to the present invention, the nucleic acid of the present invention or the expression vector of the present invention in the manufacture of a medicament for the treatment or prophylaxis of

AIDS caused by a M-trophic strain of HIV.

As the fusion protein increases the level of chemokines MIP-1α, MIP-1β, RANTES or IP-10, as well as other chemokines, the fusion protein can be seen to have a use in the treatment or prophylaxis of inflammatory disorders when it is desirable to increase or resolve an immune response, as the fusion protein can increase or resolve the inflammatory response. Suitable inflammatory disorders include bacterial peritonitis and Crohn's disease. The fusion protein of the present invention is particularly useful in the treatment of bacterial peritonitis (see Example 7).

The pharmaceutical composition of the present invention comprises any one of the compounds of the present invention (i.e. the fusion protein of the present invention, the nucleic acid molecule of the present invention or the expression vector of the present invention), with any pharmaceutically acceptable carrier, adjuvant or vehicle. Pharmaceutically acceptable carriers, adjuvants and vehicles that may be used in the pharmaceutical composition of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene- polyoxypropylene-block polymers, polyethylene glycol and wool fat.

The pharmaceutical composition of this invention may be administered orally, parenterally, by inhalation spray, or via an implanted reservoir. Preferably the pharmaceutical composition is administered orally or by injection. The pharmaceutical composition of this invention may contain any conventional non-toxic pharmaceutically-acceptable carriers, adjuvants or vehicles. The term parenteral as used herein includes subcutaneous, intracutaneous, intravenous, intramuscular, intra-articular, intrasynovial, intrasternal, intrathecal, intralesional and intracranial injection or infusion techniques.

The pharmaceutical composition may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant such as Ph. Helv or a similar alcohol.

The pharmaceutical composition of this invention may be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavouring and/or colouring agents may be added.

The pharmaceutical composition of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The present invention is now illustrated in the following examples with reference to the following figures.
Figure 1 shows a schematic representation of H-IL-6.

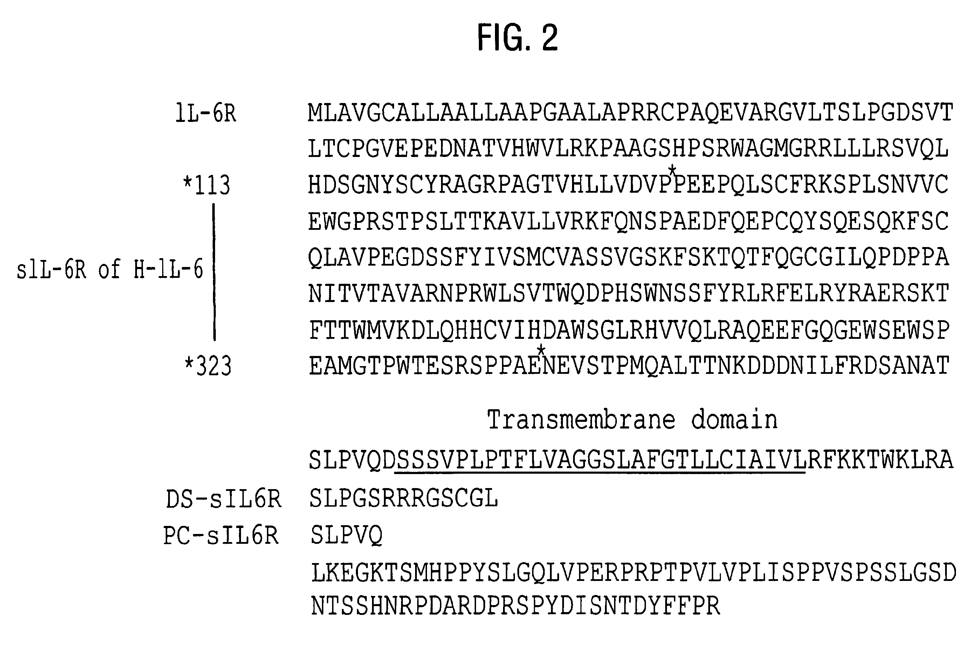

Figure 2 shows the complete IL-6R protein sequences including the membrane board form, DS-sIL-6R and PC-sIL-6R. The putative transmembrane domain is underlined.
Figure 3 shows the sequence IL-6.
Figure 4 shows the sequence DS-sIL-6R.
Figure 5 shows the sequence of IL-6 linked via a flexible linker to DS-sIL-6R (Hyper DS-sIL-6R) wherein the linker and COOH-terminal c-myc tag sequences are underlined.
Figure 6 shows the differential induction of CCR5 ligands by either DS-sIL-6R (•) or PC-sIL-6R (o) in combination with human IL-6.
Figure 7 shows the inhibition of DS-sIL-6R mediated chemokine release using (A) various monoclonal antibodies and (B) PC-sIL-6R.
Figure 8 shows the time course for chemokine induction by DS-sIL-6R (•) and PC-sIL-6R (o).
Figure 9 shows the results of the luciferase reporter assays.
Figure 10 shows the results of the EMSA analysis.
Figure 11 shows the expression and functional characterisation of Hyper DS-sIL-6R. (A) shows the level of mediator (see top left hand corner of graphs) produced and (B) shows the level of MCP-1 and RANTES produced.
Figure 12 shows the inflammatory potential of PC-sIL-6R, DS-sIL-6R, SES, SES plus PC-sIL-6R and SES plus DS-sIL-6R.

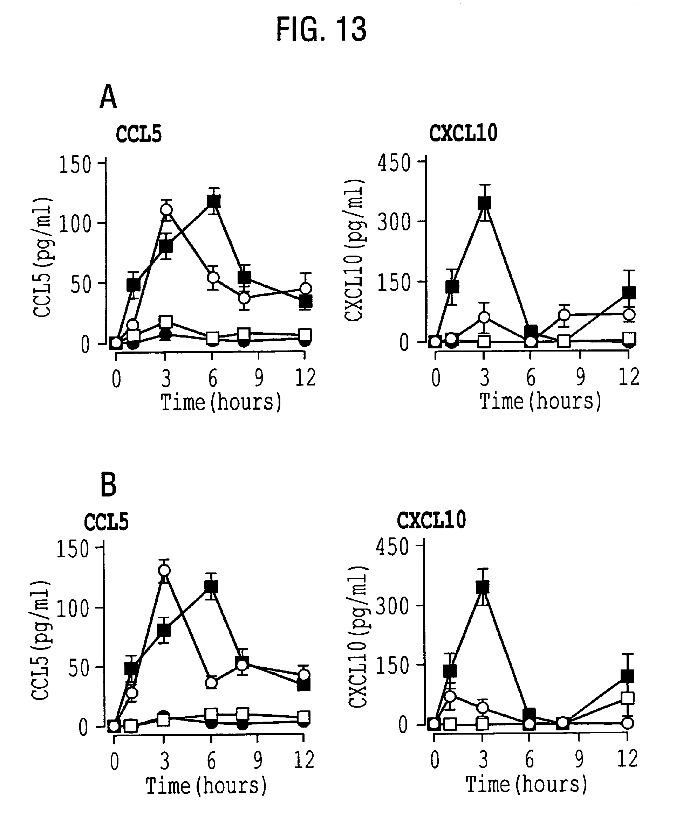

Figure 13 shows the expression of CCL5 and CXCL10 in IL-6^{+/+} and IL-6^{-/-} mice, wherein in (A) Wild type C57BL/6J (IL-6^{+/+}) and IL-6^{-/-} mice were intraperitoneally administered with SES or PBS and at the designated intervals the peritoneal cavity lavaged; (B) IL-6^{+/+} mice were administered with PBS, 150 ng/mouse sgp130 alone, SES, or SES in combination with 150-ng/mouse sgp130. At the designated time intervals the peritoneal cavity was lavaged. In both cases, CCL25 and CXCL10 levels were quantified in lavage fluid using ELISA. Values are expressed as the mean ± SEM (n = 6 mice/condition).

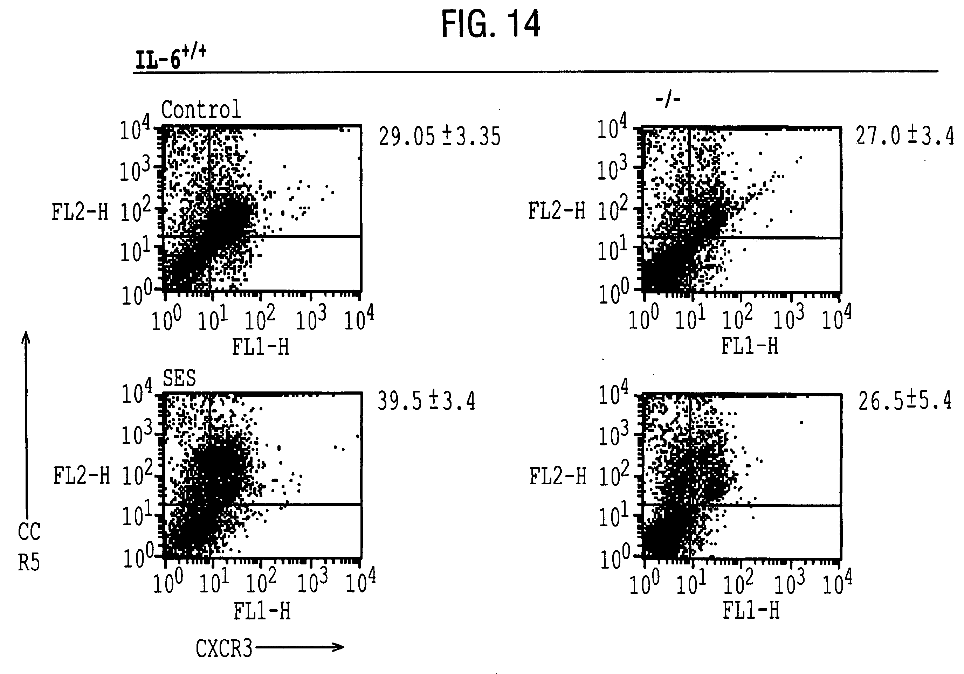

Figure 14 shows the recruitment of cells expressing CCR5 and CXCR3 in IL-6^{+/+} and IL-6^{-/-} mice, wherein IL-6^{+/+} and IL-6^{-/-} mice were administered with either PBS (control) or SES. After 12 hours the peritoneal cavity was lavaged and the recovered cells dual-labeled with antibodies for CCR5 and CXCR3 and analyzed by FACS. Quadrants were set according to autofluorescence by control antibodies and the percentage of cells lying in the upper-right segment is presented (mean ± SEM from 4-6 mice/experimental condition) along with representative scatter plots for each condition. Values represent the mean (%) ± SEM from 4-6 mice for each condition.

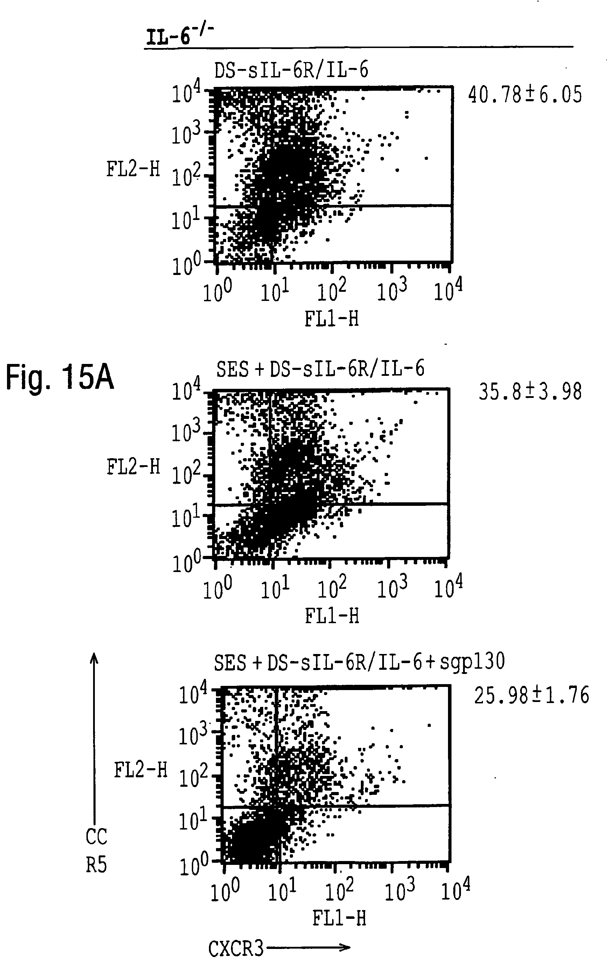

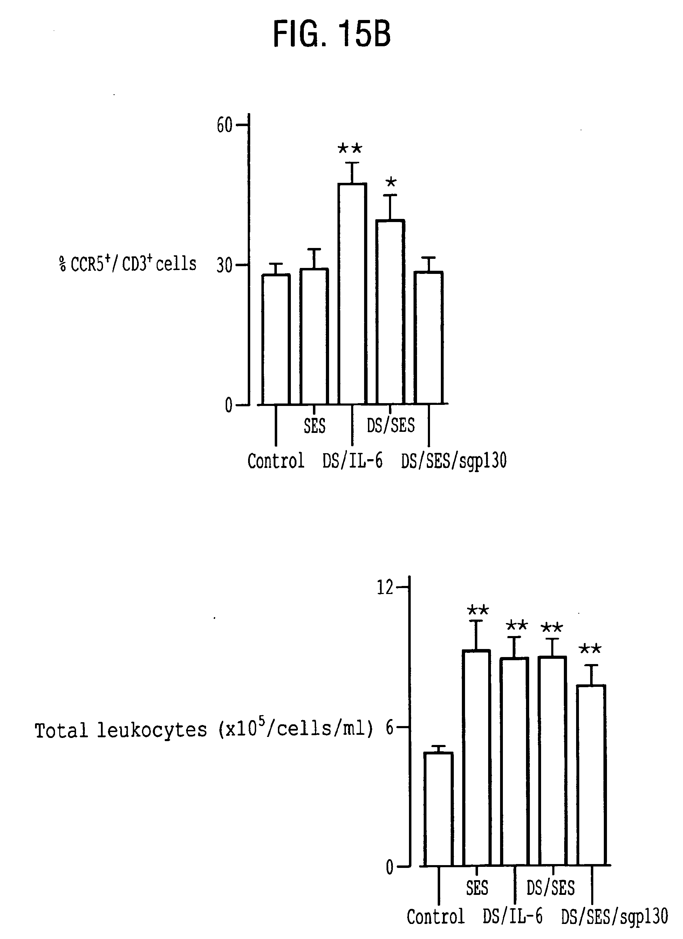

Figure 15 shows Recruitment of cells expressing CCR5 and CXCR3 in IL-6^{+/+} and IL-6^{-/-} mice. IL-6^{-/-} mice were treated with a combination of DS-sIL-6R (25 ng/mouse) and IL-6 (20 ng/mouse) (DS/IL-6) in the presence or absence of SES. Soluble gp130 (150 ng/mouse) was also included as indicated. Quadrants were set according to autofluorescence by control antibodies and the percentage of cells lying in the upper-right segment is presented (mean ± SEM from 4-6 mice/experimental condition) along with representative scatter plots for each condition (see A). (B) Percentage of recovered cells dual-labeled with antibodies for CCR5 and CD3 (upper graph). Values represent the mean (%) ± SEM from 4-6 mice for each condition. Total leukocyte influx for each experimental condition (lower graph). Values represent the mean ± SEM from 4-6 mice for each condition.

### Examples

### Materials and Methods

### Reagents

Baculovirus expressed sIL-6R isoforms were obtained as previously described (Horicuhi et al., Immunology 95, 360-369, 1994). Monoclonal anti-IL-6 and anti-sIL-6R (mAb-226 and mAb-227 respectively) were purchased from R & D systems. Anti-DS-sIL-6R (mAb-2F3) was raised against the unique COOH-terminal sequence of DS-sIL-6R (Horiuchi *et al.,* (*supra*)).

### Isolation and culture of human peritoneal mesothelial cells

Human peritoneal mesothelial cells (HPMC) were isolated by serial tryptic (0.1 % w/v trypsin: 0.02% w/v EDTA) digestion of omental tissue from consenting patients undergoing elective abdominal surgery (Topley et al., American. J. Pathology, 142, 1876-1886, 1993). Cells were cultured in Earle's buffered 199 medium containing 10% (v/v) fetal calf serum, 2 mM L-glutamine, 100 U/ml penicillin, 100 µg/ml streptomycin, 5µg/ml transferrin, 5µg/ml insulin and 0.4µg/ml hydrocortisone (Life Technologies or Sigma) at 37°C in a humidified 5% CO₂ atmosphere. Prior to experimentation, HPMC monolayers were growth arrested in the absence of serum. Under these conditions HPMC remain in a viable and quiescent state for up to 96 hours (Topley *et al.,* 1993 *(supra)).* Stimulations were performed in the absence of serum and on cells no older than the second passage.

### Determinator of inflammatory mediator concentrations

Inflammatory mediator concentrations were quantified using sandwich ELISA techniques. Human MCP-1 levels were determined using a matched antibody pair OptEIA kit from Pharmingen, Becton-Dickinson. Human RANTES was quantified with appropriate matched antibody pairs from R & D Systems (mAb678/NAF278) while human MIP-1α, MIP-1β and eotaxin were analyzed using Amersham BIOTRAK ELISA kits.

### Luciferase reporter assays

Luciferase-linked RANTES promoter constructs bearing either the complete promoter sequence or mutations within known transcription factor binding motifs were obtained from Professor H. Moriuchi, University of Nagasaki (Moriuchi et al., J. Immunol. 159, 5441-5449, 1997). Briefly, HPMC (1 x 10⁴ cells/6 well microtite plate) were cultured in M199 medium containing 10% FCS until they had reached a confluency of 60-70%. The monolayer was washed and cells transfected overnight with individual luciferase-linked promoter constructs (0.5-1.0µg). Transient transfection was performed using a standard calcium phosphate precipitation technique (Graham and van de Eb, Virology, 52, 456-458, 1973). Once the HPMC had recovered, cells were stimulated for 24 hours as indicated in example 4. Cell lysates were prepared and luciferase activity determined by luminometry using a commercial luciferase assay kit (Promega).

### Preparation of nuclear extracts

Nuclear extracts were prepared from HPMC using a rapid technique for the extraction of DNA binding proteins. Briefly, cells were harvested in ice cold PBS (pH7.4) and pelleted by centrifugation. Cells were resuspended in cold buffer A (10 mM HEPES-KOH (pH 7.9), 1.5 mM MgCl₂, 0.2 mM EDTA, 0.3mM DTT, 0.2mM PMSF) and incubated on ice for a further 20 minutes. Cellular debris was pelleted by centrifugation and the supernatant stored at -80°C until required.

### Electrophoretic mobility shift assay (EMSA) and supershift

EMSA were performed as described previously (Zhang et al., J. Biol. Chem. 272, 30607-30609, 1997). Oligonucleotides containing consensus motifs for NF-kB (5'-GATCCATGGGGAATTCCCC-3' & 5'-CATGGGGAATTCCCCATGGA-3'), STAT-3 (SIE-m67 5'CGACATTTCCCGTAAATCG-3' & 5'-CGACGATTTACGGGAAATG-3') and C/EBP (5' GACGTCACATTGCACAATCTTAA-3' & 5'-TATTAAGATTGTGCAATGTGACG-3') binding were annealed for use in EMSA. These double-stranded fragments were radiolabelled with [α³²P]-dTTP using the Klenow fragment of DNA polymerase I. Nuclear extracts were incubated with the labeled consensus sequence and DNA-protein interactions resolved by electrophoresis on a 6% polyacrylamide gel.

### Example 1

### Differential induction of CCRS-ligands

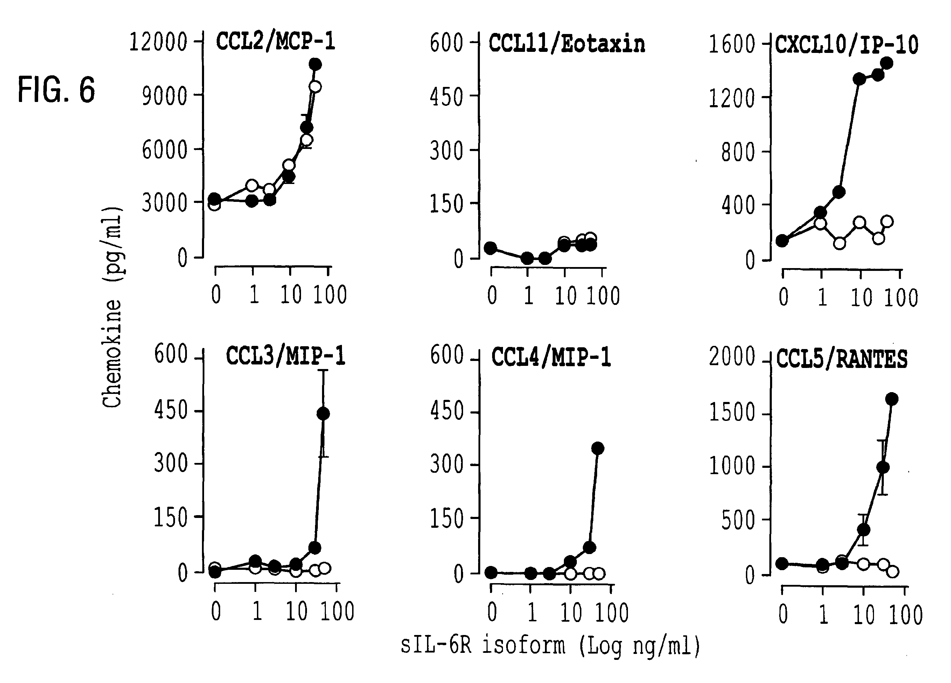

Human peritoneal mesothelial cells were growth arrested for 48 hours prior to stimulation with various concentrations (0-50 ng/ml) of either DS-sIL-6R(•) or PC-sIL-6R (o) in combination with 10 ng/ml human IL-6. Following an overnight incubation, cell-free supernatants were collected and chemokine production determined using specific ELISA. Values represent the mean ± SEM from 4 independent experiments. Data shows that DS-sIL-6R, in the presence of IL-6 induces expression of CCL3 (MIP-1α), CCL4 (MIP-1β), CCL5 (RANTES) and CXCL10 (IP-10) while PC-sIL-6R has no effect (see figure 6). The induction of CCL2 (MCP-1) and CCL11 (Eotaxin) are presented as positive and negative controls respectively.

### Example 2

### Inhibition of DS-sIL-6R mediated chemokine release

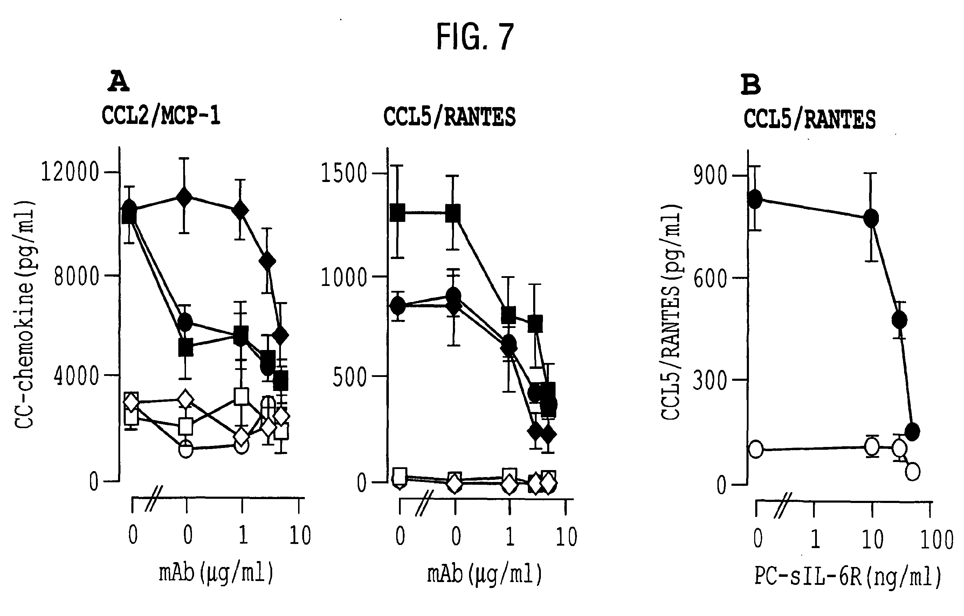

Growth-arrested human peritoneal mesothelial cells were stimulated (24 hours) with 30 ng/ml DS-sIL-6R in combination with 10 ng/ml IL-6. Release of CCL2 and CCL5 were quantified using ELISA. Figure 7A shows that human peritoneal mesothelial cells were growth-arrested for 48 hours prior to treatment. Cells treated with the various monoclonal antibodies (0-5µg/ml) in the presence (filled symbols) or absence (open symbols) of 10 ng/ml IL-6 + 20 ng/ml DS-sIL-6R. Cells treated with MAb 206 (anti IL-6) are shown as filled squares. Cells treated with MAb 227 (anti soluble IL-6) are shown as filled circles. Cells treated with MAb 2F3 (against the carboxy terminus of DS-sIL-6R) are shown as filled diamonds. Conditioned medium was harvested 24 hours later and CCL2/MCP-1 and CCL5/RANTES levels quantified using ELISA. Values represent the mean ± SEM from 4 independent experiments.

Figure 7B shows specific blockade of RANTES production by PC-sIL-6R. DS-sIL-6R mediated RANTES release was monitored in the presence of increasing concentrations of PC-sIL-6R (0-50 ng/ml) (•). Secretion of RANTES in response to PC-sIL-6R and lo ng/ml IL-6 is shown as a control (o). Values represent the mean ± SEM from 4 independent experiments. In other experiments RANTES release was blocked by the inclusion of soluble gp130, Hyper-IL-6.

### Example 3

### Time course for chemokine induction

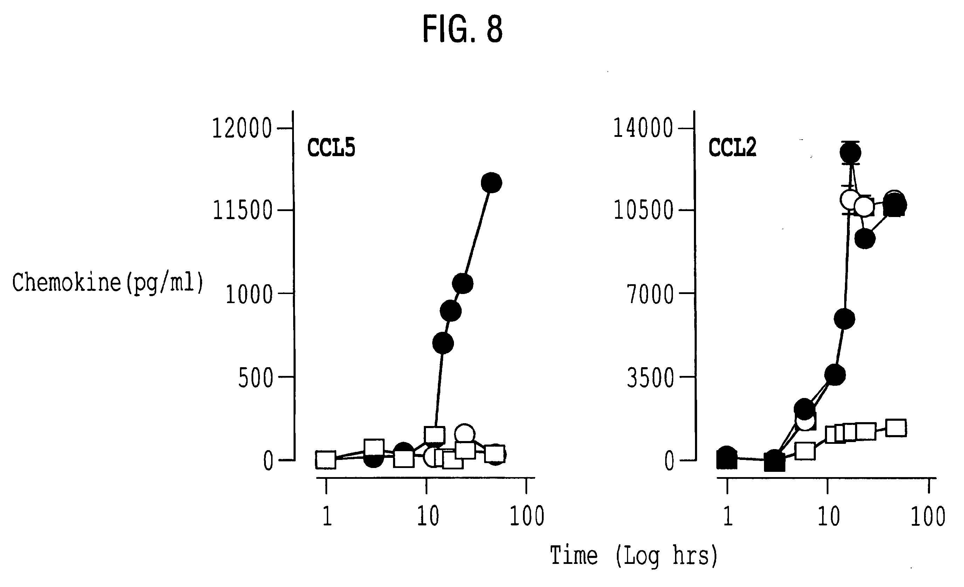

Growth-arrested HPMC were stimulated with 50 ng/ml DS-sIL-6R (•) or PC-sIL-6R (o) in the presence of 10 ng/ml IL-6. Unstimulated cells are shown for comparison (open square box). At set time intervals cell-free supernatants were collected and the release of CCL2 (MCP-1) and CCL5 (RANTES) determined. Values represent the mean ± SEM from 4 independent experiments. Data shows that although CCL2 production is detectable after 3-4 hours stimulation by sIL-6R, the DS-sIL-6R mediated release of CCL5 is not observed until 15 hours after the initial stimulation (see Figure 8). These findings suggest that the regulation of expression of CCL5 is distinct from that of CCL2.

### Example 4

### Luciferase reporter assays

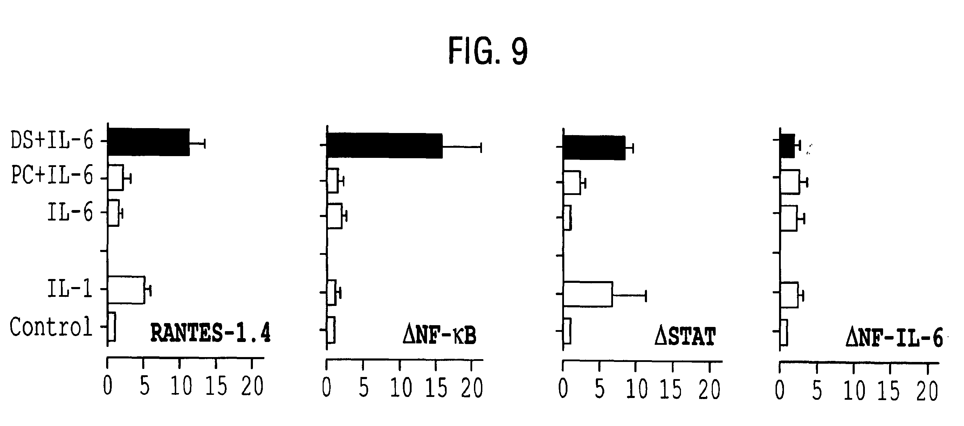

Luciferase reporter constructs containing a 1.4 kb RANTES promoter fragment (RANTES-1.4) or various mutations within known transcription factor consensus sequences (ΔNF-kB, ΔSTAT and ΔNF-IL-6) (Moriuchi *et al.,* 1997 (*supra*)). were transiently transfected into human peritoneal mesothelial cells. After recovery, cells were stimulated with either 100 pg/ml IL-1β, 10 ng/ml IL-6 alone or in combination with 50 ng/ml PC-sIL-6R (PC) or DS-sIL-6R (DS). Following a 24 hour stimulation luciferase activity was monitored. Activity of the ΔNF-IL-6 construct was confirmed using ionomycin as a control, which induced a ~40-fold increase in luciferase activity (data not shown). Values represent the fold induction obtained from experiments performed using individual primary mesothelial cell isolates and the mean ± SEM (n=4) is shown (see Figure 9). These data show that disruption of NF-IL-6 significantly blocked the DS-sIL-6R mediated induction of luciferase activity, while PC-sIL-6R and IL-6 alone showed no increase with any of the constructs above control level.

### Example 5

### EMSA Analysis

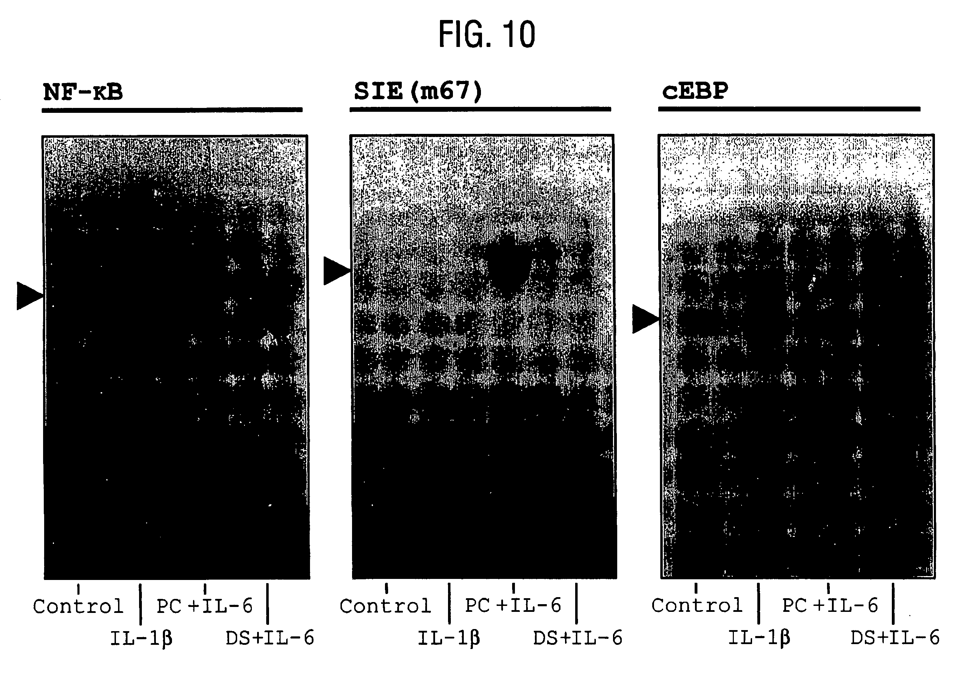

Nuclear extracts were isolated from HPMC that had been stimulated for 30 minutes with 10 pg/ml IL-1β, or 10 ng/ml IL-6 in combination with 50ng/ml PC-sIL-6R (PC) or DS-sIL-6R (DS). Consensus sequences for NF-κB, STAT-3 (SIE m67) and C/EBP (NF-IL-6) were radiolabelled and incubated with the nuclear extracts. Protein-DNA interactions were analysed by separation on a 6% polyacrylamide gel by electrophoresis and bands visualised by autoradiography (see Figure 10). Activation of NF-κB and STAT-3 were used as controls. These initial experiments emphasise that DS-sIL-6R induces enhanced binding to a C/EBP consensus sequence. Since this consensus oligonucleotide does not enable the individual C/EBP family members to be distinguished, competition EMSA and supershift approaches using specific antibody are currently being used to confirm the involvement of either NF-IL-6α (C/EBPβ) or NF-IL-6β (C/EBPδ).

### Example 6

### Expression and functional characterisation of Hyper DS-sIL-6R

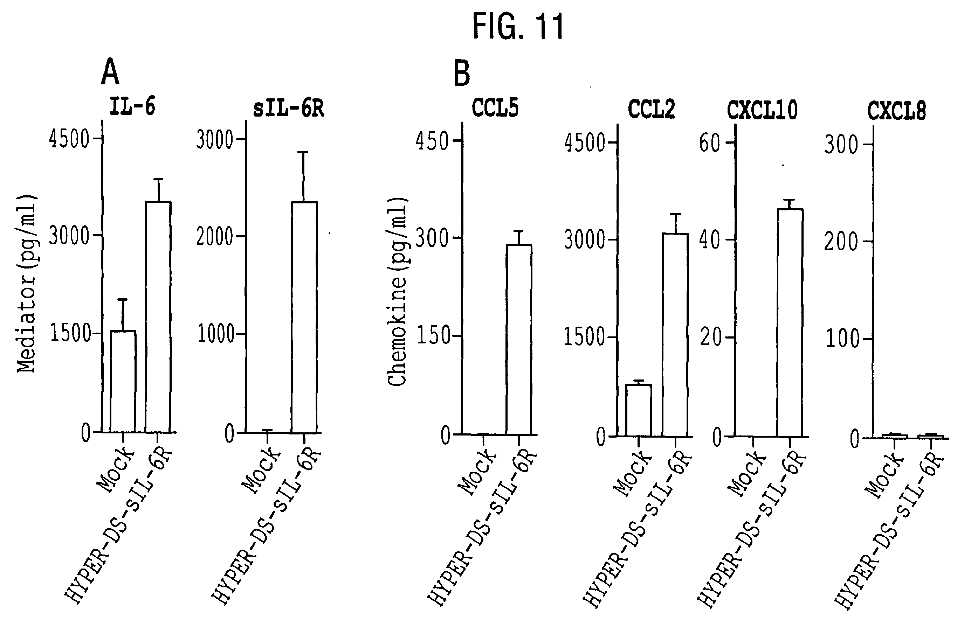

COS-7 cells were transiently transfected with either pcDNA-3 (Mock) or pcDNA-3/HYPER-DS-sIL-6R (HYPER-DS-sIL-6R) under serum-free conditions. Following a 24-hour incubation at 37°C, conditioned medium was harvested and the concentration of human IL-6 and sIL-6R quantified using specific ELISA (see Figure 11A). This conditioned medium was also added to serum-starved human synovial fibroblasts. Following a 24-hour incubation at 37°C, conditioned medium was harvested and the concentration of human CCL2/MCP-1 and CCL5/RANTES quantified using specific ELISA (see Figure 11B). Values represent the mean ± SD from a single experiment.

### Example 7

### Inflammatory Potential

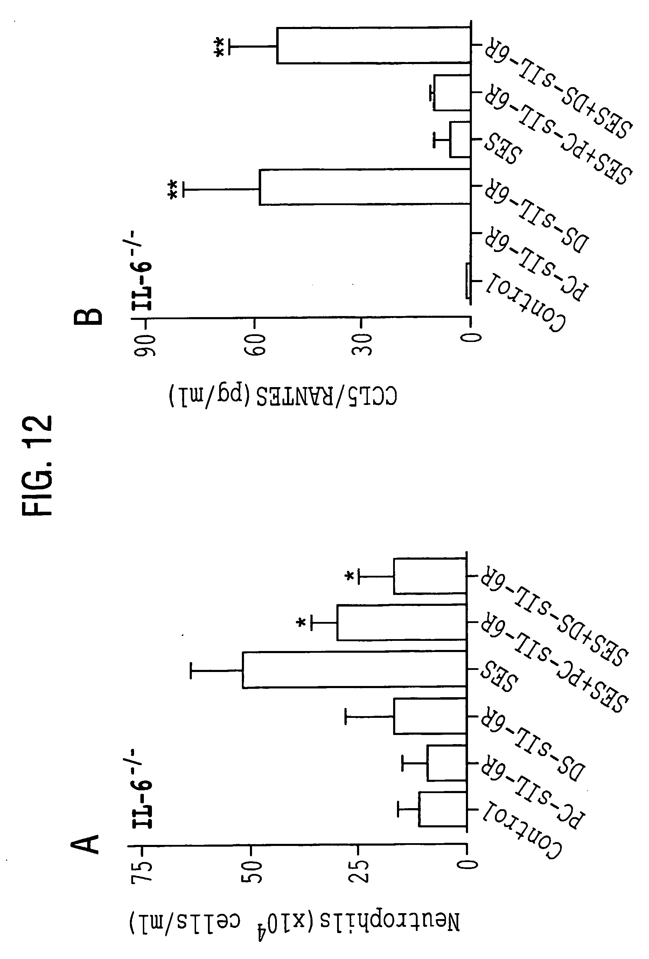

We have previously reported the characterisation of a peritoneal inflammation model in both wild type and IL-6 deficient (IL-6-/-) mice using a cell-free supernatant prepared from *Staphyloccous epidermidis* (termed SES) (Hurst et al., Immunity, 14, 705-714, 2001). Using this model, we have examined the inflammatory potential of DS- and PC-sIL-6R in IL-6 -/- mice. Mice were intraperitoneally administered with sterile PBS (control), 100 ng/mouse DS-sIL-6R and 25 ng/mouse human IL-6 (DS-sIL-6R), 100 ng/mouse PC-sIL-6R and 25 ng/mouse human IL-6 (PC-sIL-6R), SES alone (SES) or in combination with DS-sIL-6R and IL-6 or PC-sIL-6R and IL-6 (SES + DS-sIL-6R or SES + PC-sIL-6R respectively). Following a 3 hour stimulation, the peritoneal cavity was lavaged and inflammatory parameters determined. As previously reported for PC-sIL-6R (Hurst et al., Immunity, 14, 705-714, 2001) both isoforms inhibited the infiltration of neutrophilis following exposure to SES (see Figure 12 A). Levels of CCL5/RANTES were also determined in the lavage fluid and consistent with our *in vitro* data was found to be preferentially induced by the action of DS-sIL-6R + IL-6 (see Figure 12B). Values represent the mean ± SEM (n=3-4 animals/condition * = p<0.05; ** = p<0.001).

### Example 8

### Expression of CCL5 and CXCL10 in wild type (IL-6^{+/+}) and IL-6-deficient (IL-6^{-/-}) mice

The selective induction of chemokines specific for the receptors CCR5 (CCL3, CCL4, CCL5) and CXCR3 (CXCL10) by DS-sIL-6R suggests that this isoform might be important in attracting T-lymphocytes and monocytes to sites of inflammation. To test this, a recently characterized model of peritoneal inflammation was used to determine the temporal expression of CCL5 and CXCL10 (CRG-2, the murine homologue of IP-10) in IL-6^{+/+} and IL-6^{-/-} mice. This model closely resembles a bacterial-peritonitis episode typically encountered in peritoneal dialysis patients and is based on the intraperitoneal administration of a bacterial-cell free supernatant derived from *Staphylococcus epidermidis* (termed SES) (Hurst et al., 2001). As illustrated in Figure 13A, the profiles for CCL5 and CXCL10 expression seen in SES challenged IL-6^{-/-} mice were considerably altered from those encountered in IL-6^{+/+} mice. These modified patterns of expression were the result of sIL-6R signaling since blockade of sIL-6R activity in IL-6^{+/+} mice with soluble gp130 (sgp130) converted the profile of CCL5 and CXCL10 release seen in SES-treated IL-6^{+/+} mice to that observed in IL-6^{-/-} mice (Figure 13B).

### Example 9

### Functional characterization of DS-sIL-6R activity in IL-6^{-/-} mice

To test whether the modified pattern of CCL5 and CXCL10 release in IL-6^{-/-} mice has a direct affect on leukocyte recruitment, IL-6^{+/+} and IL-6^{-/-} mice were challenged with SES and FACS staining performed on cells lavaged from the peritoneal cavity using antibodies against murine CCR5 and CXCR3 (Figure 14). Treatment of IL-6^{+/+} mice with SES induced an increase in the cell types bearing CCR5 and CXCR3, however this infiltration was impaired in SES treated IL-6^{-/-} mice (Figure 14). Infiltration of CCR5⁺/CXCR3⁺ cells were also assessed in IL-6^{-/-} mice that had been challenged with SES in the presence of DS-sIL-6R and IL-6 (Figure 15A). As predicted by the chemokine data, reconstitution of IL-6 signaling with DS-sIL-6R restored the influx of CCR5⁺/CXCR3⁺ positive cells to levels encountered in IL-6^{+/+} mice. Increased recruitment of these leukocytes was specific to DS-sIL-6R and was blocked by sgp130 (Figure 15A). Similar data was obtained for cell populations dual labeled with anti-CD3 and anti-CCR5 (Figure 15B). These responses were specific for DS-sIL-6R and were not induced via PC-sIL-6R (Figure 15A & B). In all cases, administration of IL-6^{-/-} mice with SES induced significant increases in the total number of leukocytes recruited to the peritoneal cavity (Figure 15B). Consequently, IL-6 appears to influence the attraction of leukocytes expressing CXCR3 and CCR5 through binding DS-sIL-6R and emphasizes that the biological properties of DS-sIL-6R are clearly distinct from those of PC-sIL-6R.

Interleukin-6 (IL-6) has been described as having both pro- and anti-inflammatory effects. In terms of its protective properties, IL-6 appears to moderate the extent of an inflammatory response through its ability to block pro-inflammatory cytokine expression and by promoting release of IL-1 receptor antagonist and the soluble p55 TNFα-receptor (Schindler et al., Blood 75, 40-44, 1990; Tilg et al., Blood, 83, 113-118, 1994 and Xing et al., J. Clin. Invest. 101, 311-320, 1998). Interleukin-6 may also influence leukocyte recruitment, since accumulation of neutrophils at sites of infection or inflammation is suppressed by its action (Ulich et al., Am. Pathol.138, 1097-1102,1991; Barton et al., Infect. Immunol. 61 1496-1500, 1993 and Xing et al., J. Clin. Invest. 101, 311-320, 1998). Indeed, exposure of IL6-deficient (IL6^{-/-}) mice to an endotoxin aerosol results in a significantly greater number of neutrophils in broncho-alveolar lavage fluid than wild-type (IL-6^{+/+}) animals (Xing et al., J. Clin. Invest. 101, 311-320, 1998).

Central to the regulation of IL-6-mediated responses is the presence of a soluble IL6 receptor (sIL-6R), which forms a ligand-receptor complex that allows IL-6 responsiveness in cell types lacking expression of the cognate IL-6 receptor (IL-6R) (Jones et al., FASEB. J. 15, 43-58, 2001). This [sIL6R/IL6] complex has the capacity to activate cells that do not normally respond to IL-6 through interaction with the ubiquitously expressed signal-transducing element for the IL-6-family of cytokines, gp130 (Jones et al., FASEB. J. 15, 43-58, 2001). It is through this mechanism that the sIL-6R can induce expression of certain chemokines (IL-8, MCP-1, MCP-3) and adhesion molecules (ICAM-1 and VCAM-1) (Romano et al., Immunity 6, 315-325, 1997; Modur et al., J. Clin. Invest. 100, 2752-2756,1997 and Klouche et al., J. Immunol. 163, 4583-4589, 2000). Recently, we have proposed that sIL-6R release acts as an important intermediary in the resolution of inflammation and supports the transition between the acute predominantly neutrophilic stage of an infection, and the more sustained mononuclear cell influx. Consequently, sIL6R mediated signaling might contribute to the previously described effects of IL-6 on leukocyte recruitment.

This present application indicates that PC-sIL-6R and DS-sIL-6R regulate the expression of certain CC-chemokines. Although both forms were found to activate MCP-1 expression, only DS-sIL-6R could elicit release of the CCR5 ligands RANTES, MIP-1α and MIP-1β. This means that DS-sIL-6R may serve a prominent role in mononuclear leukocyte (T-lymphocytes and monocytes) recruitment and activation (Moser & Loetscher, 2001). Indeed, CCR5 is a marker for Th1 cellular responses which are typically associated with certain inflammatory diseases (Qin et al., J. Clin. Invest. 101: 746-754, 1998; Sallusto et al., J. Exp. Med. 187: 875-883, 1998;). The specific up-regulation of RANTES, MIP-1α and MIP-1β by DS-sIL-6R suggests that this sIL-6R isoform might be involved in controlling the homing of this T-cell subset to these sites. Recently, however it has been reported that IL-6 acts on T-cells to inhibit their differentiation to Th1 cells (Diehl et al., Immunity 13, 805-815, 2000). In this respect, the release of DS-sIL-6R from T cells (Horiuchi et al., Immunology 95, 360-369,1994) may also serve another role in the control of Th1 polarization, since sIL-6R has been suggested to act as an antagonistic molecule that enhances the inhibitory capacity of soluble gp130 (Müller-Newen et al., Eur. J. Biochem. 236, 837-842, 1998). Thus, by mopping up any free IL-6, DS-sIL-6R may prevent IL-6 acting directly on the T-cell subset to influence their phenotype.

To date very little is known about the inflammatory events controlled by the IL-6-mediated activation of NF-IL-6. In general, IL-6 is thought to active two set of genes through activation of STAT-3 and NF-IL-6 family of transcription factors. Thus in the case of the acute phase response, NF-IL-6 has been reported to regulate expression of class 1 acute phase proteins (e.g., C-reactive protein and serum amyloid), while Class 2 acute phase genes such as fibrinogen are controlled via STAT-3 (Zhang et al., J. Biol. Chem. 272, 30607-30609, 1997). Evidence presented here clearly shows that although both isoforms of sIL-6R are capable of activating STAT-3; DS-sIL-6R possesses the unique ability to regulate NF-IL-6 transactivation. It is this differential activation of NFIL-6 which appears to coordinate expression of RANTES, and presumably that of MIP-1α and MIP-1β. In this respect, time dependent analysis of MCP-1, RANTES, MIP-1α and MIP-1β expression revealed that MCP-1 was induced considerably earlier than that of RANTES, MIP-1α and MIP-1β. This apparent differential control of CC-chemokine expression may be attributed to the individual regulation of STAT-3 and NF-IL-6 transactivation by the sIL-6R isoforms.

Analysis of clinical samples have previously shown that sIL-6R levels are independently controlled during disease progression by both differential mRNA splicing and proteolytic (shedding) cleavage (Horiuchi et al., Immunology, 95, 360-369, 1998 and Jones et al., FASEB. J. 15, 43-58, 2001). Consequently, DSsIL-6R and PC-sIL-6R may individually contribute to the overall properties of this soluble cytokine receptor. Recently, we have analyzed the expression profile for sIL-6R in peritoneal effluents obtained from patients on continuous ambulatory peritoneal dialysis (CAPD) with overt clinical peritonitis. Through analysis of these samples, it was shown that although total sIL-6R levels were raised within the first 24-48 hours following infection, DS-sIL-6R concentrations were only significantly increased on day 3 of the inflammation. This implies a role for DS-sIL-6R during the latter stages of disease. Through analysis of clinical peritonitis samples, we have seen that expression of RANTES, MIP-1α and MIP1β occurs much later (day 4) in the course of infection than that of MCP-1, which peaks on day 1 and returns to baseline by day 3 (data not shown). Indeed, the specific induction of RANTES, MIP-1α and MIP-1β by DS-sIL-6R not only infers that these chemokines are expressed later in the inflammatory process, but that their action is distinct from that of other CCchemokines such as MCP-1. In this respect, it has been shown that all of the CCR5-ligands are capable of activating IL-12 production by dendritic cells, whilst MCP-1-4 has been reported to suppress IL-12 production by gamma-IFN activated mononuclear leukocytes (Aliberti et al., Nature Immunol. 1, 83-87, 2000; Braun et al., J. Immunol, 164, 3009-3017, 2000). Consequently, differential expression of CCchemokines by the sIL-6R isoforms can contribute not only to the recruitment of distinct mononuclear leukocyte sub-populations, but can also influence the expression profiles of other mediators that participate in resolution of inflammation.

As all 3 chemokines as well as M-trophic strains of HIV bind CCR5, high levels of MIP-1α, MIP-1β and RANTES compete with the virus for CCR5 binding and effectively suppress HIV entry. Consequently, any factor capable of redressing the balance of this competition in the favour of the chemokine can be useful as an HIV therapy. The use of the fusion protein of the present invention is therefore useful in the treatment of any disease wherein the infectious agent binds to CCR5, especially M-trophic strains of HIV.

## Claims

1. A fusion protein comprising a functional IL-6 molecule and a functional DS-sIL-6R molecule, wherein the protein increases the expression of one or more of MIP-1α, MIP-1β, RANTES or IP-10.

2. The fusion protein of claim 1, wherein the functional IL-6 molecule and the functional DS-sIL-6R molecule are linked together by a linker.

3. The fusion protein according to claim 2 wherein the linker comprises the sequence RGGGGSGGGGSVE.

4. The fusion protein according to any one of the preceding claims, wherein the functional IL-6 molecule comprises the sequence shown in SEQ ID NO. 9.

5. The fusion protein according to any one of the preceding claims, wherein the functional DS-sIL-6R molecule comprises residues 113 to 364 of SEQ ID NO. 10.

6. The fusion protein according to any one of the preceding claims, wherein the functional DS-sIL-6R molecule comprises the sequence of SEQ ID NO. 10.

7. The fusion protein according to any one of the preceding claims, comprising one or more functional IL-6 molecules and one or more functional DS-sIL-6R molecules.

8. The fusion protein according to any one of claims 1 to 6, which comprises one functional IL-6 molecule and one functional DS-sIL-6R molecule.

9. The fusion protein according to any one of the preceding claims, wherein the fusion protein increases the expression of MIP-1α, MIP-1β and RANTES.

10. The fusion protein according to any one of claims 1 to 8, wherein the fusion protein increases the expression of MIP-1α, MIP-1β, RANTES and IP-10.

11. The fusion protein according to any one of the preceding claims, wherein the fusion protein increases the expression of MIP-α, MIP-β or RANTES by at least 5 fold.

12. A nucleic acid molecule encoding the fusion protein of any one of the preceding claims.

13. An expression vector comprising the nucleic acid molecule of claim 12.

14. The vector of claim 13, which comprises a promoter and other regulatory sequences in order to obtain expression of the nucleic acid molecule.

15. A host cell transformed with the vector of claim 13 or claim 14.

16. A method for producing the fusion protein according to any one of claims 1 to 10 comprising expressing the nucleic acid molecule of claim 12 in a suitable host cell and isolating the protein.

17. The method of claim 16, comprising transforming a host cell with the vector according to claim 13 or claim 14, culturing the host cell under suitable conditions for the production of the fusion protein according to any one of claims 1 to 11, and isolating the fusion protein.

18. A screening method for identifying agonists or antagonists of the fusion protein according to any one of claims 1 to 11, comprising testing a candidate molecule in order to determine if it affects the function of the fusion protein.

19. A pharmaceutical composition comprising the fusion protein according to any one of claims 1 to 11 and a pharmaceutically acceptable carrier, diluent or vehicle.

20. A pharmaceutical composition comprising the nucleic acid of claim 12 of the expression vector of claim 13 or claim 14, and a pharmaceutically acceptable carrier, diluent or vehicle.

21. The fusion protein according to any one of claims 1 to 11, the nucleic acid according to claim 12 or the expression vector according to claim 13 or claim 14, for use in therapy.

22. Use of the fusion protein according to any one of claims 1 to 11, the nucleic acid according to claim 12, or the expression vector according to claim 13 or claim 14, in the manufacture of a medicament for the prophylaxis or treatment of bacterial peritonitis.

23. Use of the fusion protein according to any one of claims 1 to 11, the nucleic acid according to claim 12, or the expression vector according to claim 13 or claim 14, in the manufacture of a medicament for the prophylaxis or treatment of AIDS caused by a M-trophic strain of HIV.

## Patentansprüche

1. Fusionsprotein, das ein funktionelles IL-6-Molekül und ein funktionelles DS-sIL-6R-Molekül umfasst, wobei das Protein die Expression von ein oder mehreren von MIP-1α, MIP-1β, RANTES oder IP-10 steigert.

2. Fusionsprotein nach Anspruch 1, wobei das funktionelle IL-6-Molekül und das funktionelle DS-sIL-6R-Molekül über einen Linker miteinander verknüpft sind.

3. Fusionsprotein nach Anspruch 2, wobei der Linker die Sequenz RGGGGSGGGGSVE umfasst.

4. Fusionsprotein nach einem der vorhergehenden Ansprüche, wobei das funktionelle IL-6-Molekül die in SEQ ID NO. 9 gezeigte Sequenz umfasst.

5. Fusionsprotein nach einem der vorhergehenden Ansprüche, wobei das funktionelle DS-sIL-6R-Molekül die Reste 113 bis 364 von SEQ ID NO. 10 umfasst.

6. Fusionsprotein nach einem der vorhergehenden Ansprüche, wobei das funktionelle DS-sIL-6R-Molekül die Sequenz von SEQ ID NO. 10 umfasst.

7. Fusionsprotein nach einem der vorhergehenden Ansprüche, welches ein oder mehr funktionelle IL-6-Moleküle und ein oder mehr funktionelle DS-sIL-6R-Moleküle umfasst.

8. Fusionsprotein nach einem der Ansprüche 1 bis 6, welches ein funktionelles IL-6-Molekül und ein funktionelles DS-sIL-6R-Molekül umfasst.

9. Fusionsprotein nach einem der vorhergehenden Ansprüche, wobei das Fusionsprotein die Expression von MIP-1α, MIP-1β und RANTES steigert.

10. Fusionsprotein nach einem der Ansprüche 1 bis 8, wobei das Fusionsprotein die Expression von MIP-1α, MIP-1β, RANTES und IP-10 steigert.

11. Fusionsprotein nach einem der vorhergehenden Ansprüche, wobei das Fusionsprotein die Expression von MIP-α, MIP-β oder RANTES um wenigstens das Fünffache steigert.

12. Nukleinsäuremolekül, welches das Fusionsprotein nach einem der vorhergehenden Ansprüche codiert.

13. Expressionsvektor, umfassend das Nukleinsäuremolekül nach Anspruch 12.

14. Vektor nach Anspruch 13, welcher einen Promotor und andere regulatorische Sequenzen zur Expression des Nukleinsäuremoleküls umfasst.

15. Wirtszelle, welche mit dem Vektor nach Anspruch 13 oder Anspruch 14 transformiert ist.

16. Verfahren zur Herstellung des Fusionsproteins nach einem der Ansprüche 1 bis 10, umfassend die Expression des Nukleinsäuremoleküls nach Anspruch 12 in einer geeigneten Wirtszelle und Isolieren des Proteins.

17. Verfahren nach Anspruch 16, umfassend Transformation einer Wirtszelle mit dem Vektor nach Anspruch 13 oder Anspruch 14, Kultivieren der Wirtszelle unter zur Produktion des Fusionsproteins nach einem der Ansprüche 1 bis 11 geeigneten Bedingungen, und Isolieren des Fusionsproteins.

18. Screening-Verfahren zur Identifizierung von Agonisten oder Antagonisten des Fusionsproteins nach einem der Ansprüche 1 bis 11, umfassend Testen eines Kandidatenmoleküls zur Bestimmung, ob es die Funktion des Fusionsproteins beeinflusst.

19. Pharmazeutische Zusammensetzung, umfassend das Fusionsprotein nach einem der Ansprüche 1 bis 11 und einen pharmazeutisch verträglichen Träger, ein pharmazeutisch verträgliches Verdünnungsmittel oder ein pharmazeutisch verträgliches Vehikel.

20. Pharmazeutische Zusammensetzung, umfassend die Nukleinsäure nach Anspruch 12 oder den Expressionsvektor nach Anspruch 13 oder Anspruch 14 und einen pharmazeutisch verträglichen Träger, ein pharmazeutisch verträgliches Verdünnungsmittel oder ein pharmazeutisch verträgliches Vehikel.

21. Fusionsprotein nach einem der Ansprüche 1 bis 11, Nukleinsäure nach Anspruch 12 oder Expressionsvektor nach Anspruch 13 oder Anspruch 14 zur therapeutischen Verwendung.

22. Verwendung des Fusionsproteins nach einem der Ansprüche 1 bis 11, der Nukleinsäure nach Anspruch 12 oder des Expressionsvektors nach Anspruch 13 oder Anspruch 14 zur Herstellung eines Medikaments zur Vorbeugung oder Behandlung von bakterieller Peritonitis.

23. Verwendung des Fusionsproteins nach einem der Ansprüche 1 bis 11, der Nukleinsäure nach Anspruch 12 oder des Expressionsvektors nach Anspruch 13 oder Anspruch 14 zur Herstellung eines Medikaments zur Vorbeugung oder Behandlung von AIDS mit einem M-trophen HIV-Stamm als Ursache.

## Revendications

1. Protéine de fusion comprenant une molécule d'IL-6 fonctionnelle et une molécule de DS-sIL-6R fonctionnelle, la protéine augmentant l'expression d'un ou plusieurs parmi MIP-1α, MIP-1β, RANTES ou IP-10.

2. Protéine de fusion selon la revendication 1, dans laquelle la molécule d'IL-6 fonctionnelle et la molécule de DS-sIL-6R fonctionnelle sont liées ensemble par un lieur.

3. Protéine de fusion selon la revendication 2, dans laquelle le lieur comprend la séquence RGGGGSGGGGSVE.

4. Protéine de fusion selon l'une quelconque des revendications précédentes, dans laquelle la molécule d'IL-6 fonctionnelle comprend la séquence présentée dans SEQ ID N° 9.

5. Protéine de fusion selon l'une quelconque des revendications précédentes, dans laquelle la molécule de DS-sIL-6R fonctionnelle comprend les résidus 113 à 364 de SEQ ID N° 10.

6. Protéine de fusion selon l'une quelconque des revendications précédentes, dans laquelle la molécule de DS-sIL-6R fonctionnelle comprend la séquence de SEQ ID N° 10.

7. Protéine de fusion selon l'une quelconque des revendications précédentes, comprenant une ou plusieurs molécules d'IL-6 fonctionnelles et une ou plusieurs molécules de DS-sIL-6R fonctionnelles.

8. Protéine de fusion selon l'une quelconque des revendications 1 à 6, qui comprend une molécule d'IL-6 fonctionnelle et une molécule de DS-sIL-6R fonctionnelle.

9. Protéine de fusion selon l'une quelconque des revendications précédentes, la protéine de fusion augmentant l'expression de MIP-1α, MIP-1β et RANTES.

10. Protéine de fusion selon l'une quelconque des revendications 1 à 8, la protéine de fusion augmentant l'expression de MIP-1α, MIP-1β, RANTES et IP-10.

11. Protéine de fusion selon l'une quelconque des revendications précédentes, la protéine de fusion augmentant d'au moins 5 fois l'expression de MIP-α, MIP-β ou RANTES.

12. Molécule d'acide nucléique codant la protéine de fusion selon l'une quelconque des revendications précédentes.

13. Vecteur d'expression comprenant la molécule d'acide nucléique selon la revendication 12.

14. Vecteur selon la revendication 13, qui comprend un promoteur et d'autres séquences régulatrices afin d'obtenir l'expression de la molécule d'acide nucléique.

15. Cellule hôte transformée avec le vecteur selon la revendication 13 ou la revendication 14.

16. Procédé de production de la protéine de fusion selon l'une quelconque des revendications 1 à 10 comprenant l'expression de la molécule d'acide nucléique selon la revendication 12 dans une cellule hôte adaptée et l'isolement de la protéine.

17. Procédé selon la revendication 16, comprenant la transformation d'une cellule hôte avec le vecteur selon la revendication 13 ou la revendication 14, la culture de la cellule hôte dans des conditions adaptées pour la production de la protéine de fusion selon l'une quelconque des revendications 1 à 11 et l'isolement de la protéine de fusion.

18. Procédé de criblage pour identifier des agonistes ou des antagonistes de la protéine de fusion selon l'une quelconque des revendications 1 à 11, comprenant l'évaluation d'une molécule candidate afin de déterminer si elle affecte la fonction de la protéine de fusion.

19. Composition pharmaceutique comprenant la protéine de fusion selon l'une quelconque des revendications 1 à 11 et un support, diluant ou véhicule pharmaceutiquement acceptables.

20. Composition pharmaceutique comprenant l'acide nucléique selon la revendication 12 du vecteur d'expression selon la revendication 13 ou la revendication 14, et un support, un diluant ou un véhicule pharmaceutiquement acceptables.

21. Protéine de fusion selon l'une quelconque des revendications 1 à 11, acide nucléique selon la revendication 12 ou vecteur d'expression selon la revendication 13 ou la revendication 14, pour utilisation en thérapie.

22. Utilisation de la protéine de fusion selon l'une quelconque des revendications 1 à 11, de l'acide nucléique selon la revendication 12 ou du vecteur d'expression selon la revendication 13 ou la revendication 14, dans la fabrication d'un médicament pour la prophylaxie ou le traitement de la péritonite bactérienne.

23. Utilisation de la protéine de fusion selon l'une quelconque des revendications 1 à 11, de l'acide nucléique selon la revendication 12 ou du vecteur d'expression selon la revendication 13 ou la revendication 14, dans la fabrication d'un médicament pour la prophylaxie ou le traitement du SIDA causé par une souche M-trophique du VIH.
